# EUROPEAN PATENT SPECIFICATION

(11) **EP 4 332 219 B1**
(45) Date of publication and mention of the grant of the patent: **18.02.2026**
(21) Application number: 22807703.8
(22) Date of filing: 03.05.2022
(51) Int. Cl.: C12N 9/78, C07K 7/08, C07K 7/06, A61K 38/00, A61K 8/64, C12N 15/63, C07K 14/47, C12N 15/70, C12N 15/87

(54) **CARGO MOLECULE TRANDUCTION DOMAIN RMAD1, VARIANT THEREOF, RECOMBINANT CARGO MOLECULE, AND METHOD FOR TRANDUCING CARGO MOLECULE USING SAME**
CARGOMOLEKÜL-TRANSDUKTIONSDOMÄNE RMAD1, VARIANTE DAVON, REKOMBINANTES CARGOMOLEKÜL UND VERFAHREN ZUM TRANSDUKTIEREN EINES CARGOMOLEKÜLS DAMIT
DOMAINE DE TRANSDUCTION DE MOLÉCULE CARGO RMAD1, VARIANT DE CELUI-CI, MOLÉCULE CARGO RECOMBINÉE, ET PROCÉDÉ DE TRANSFERT DE MOLÉCULE CARGO L'UTILISANT

(30) Priority: 14.05.2021 KR 20210062397; 29.04.2022 KR 20220053247
(43) Date of publication of application: 06.03.2024
(73) Proprietor: REMEDI CO., LTD, Incheon 21990 (KR)
(72) Inventor: PARK, Chanho, Incheon 22009 (KR); CHO, Seongmin, Incheon 21986 (KR)
(74) Representative: Sander, Rolf
(86) International application number: PCT/KR2022/006340
(87) International publication number: WO 2022/240055

(56) References cited:
- WO-A1-2015/005723
- KR-A- 20040 075 236
- KR-A- 20120 026 408
- KR-A- 20150 145 132
- KR-B1- 101 476 953
- KR-B1- 102 060 411
- NAKASE IKUHIKO ET AL: "Cell-surface Accumulation of Flock House Virus-derived Peptide Leads to Efficient Internalization via Macropinocytosis", MOLECULAR THERAPY, vol. 17, no. 11, 1 November 2009 (2009-11-01), US, pages 1868 - 1876, XP093203312, ISSN: 1525-0016, DOI: 10.1038/mt.2009.192
- DERAKHSHANKHAH HOSSEIN ET AL: "Cell penetrating peptides: A concise review with emphasis on biomedical applications", BIOMEDICINE & PHARMACOTHERAPY, ELSEVIER, FR, vol. 108, 28 September 2018 (2018-09-28), pages 1090 - 1096, XP085532568, ISSN: 0753-3322, DOI: 10.1016/J.BIOPHA.2018.09.097

## Description

### BACKGROUND

### Field

The present invention relates to a cargo molecule transduction domain RMAD1 derived from human ADARB2, a variant thereof, a gene structure encoding the same, a vector, and a recombinant cargo molecule in which the cargo molecule transduction domain for use in delivering cargo molecules into a cell in therapy.

### Related Art

A protein transduction domain (PTD) or cell-penetrating peptide (CPP) is a delivery system that fuses a peptide comprising approximately 5 to 30 amino acids with high-molecular-weight materials such as proteins or nucleic acids, thus easily delivering the fused substances into living bodies, for example, mammalian cells, tissues, or blood.

Although the specific mechanism has not yet been accurately identified, a protein delivery technology has been used to deliver proteins into cells or tissues for *in vitro* or *in vivo* treatment, and various protein transduction domains are known. Bonding between the protein transduction domain and biological cargo molecules (for example, nucleic acids, proteins, peptides, small molecules, cytotoxic drugs, etc.) may be achieved through various methods such as ionic bonding and electrostatic bonding in addition to covalent bonding.

The protein transduction domain has the benefit of lower toxicity and less immune rejection compared to other deliveries such as liposomes or polymers. However, protein transduction domains that are still used clinically are rare.

Double-stranded RNA-specific editase B2 (RNA-editing deaminase-2; abbreviated as "RED2" or "ADARB2") is an enzyme encoded by the ADARB2 gene in humans. This enzyme lacks editing activity, preventing other ADAR enzymes from binding to their targets *in vitro* and reducing the efficiency of these enzymes. ADARB2 protein may bind not only dsRNA but also ssRNA. ADARB2 is a member of the double-stranded RNA (dsRNA) adenosine deaminase family of RNA editing enzymes. Adenosine deamination of pre-mRNA results in changes in the amino acid sequence of the gene product, which differ from that predicted by the genomic DNA sequence.

However, there is currently no known possibility that some sequences of the ADARB2 protein may be used as cargo molecule transduction domains.

NAKASE IKUHIKO ET AL: "Cell-surface Accumulation of Flock House Virus-derived Peptide Leads to Efficient Internalization via Macropinocytosis", MOLECULAR THERAPY, vol. 17, no. 11, 1 November 2009 (2009-11-01), pages 1868-1876, XP093203312, discloses an Arginine-rich cell-penetrating peptides (CPPs), including human immunodeficiency virus type 1 (HIV-1) Tat (48-60) and oligoarginines, have been applied as carriers for delivery of cargo molecules. DERAKHSHANKHAH HOSSEIN ET AL: "Cell penetrating peptides: A concise review with emphasis on biomedical applications", BIOMEDICINE & PHARMACOTHERAPY, ELSEVIER, FR, vol. 108, 28 September 2018 (2018-09-28), pages 1090-1096, XP085532568, discloses CPPs to transport across cellular membrane which can employ as an appropriate carrier for various cargos include nucleic acid, proteins, SiRNA, therapeutic agents, nanoparticles and so on.

### SUMMARY

The subject matter of the present invention are definded in the independent claims. Embodiments are defined in the dependent claims. An embodiment of the present invention provides a penetrating cargo molecules into cells or tissues by penetrating the cargo molecules into the cells or tissues with high efficiency and using a cargo molecule transduction domain with no or minimal side effects when used in the human body, a recombinant cargo molecule fused with the cargo molecule transduction domain, and the cargo molecule transduction domain.

As a result of selecting and testing numerous human-derived candidate peptides, the present inventors found that a peptide consisting of 15 amino acids of CKSKRRRRRRSKRKD derived from human ADARB2 proteins (hereinafter referred to as "RMAD1" in an embodiment of the present invention) or peptides in which some amino acids among them are deleted, substituted, and/or added was/were able to smoothly penetrate polymers such as proteins and nucleic acids into the living body such as cells, tissues, and blood, and that the RMAD1 or its variant peptide had significantly superior cell and tissue penetration ability compared to the HIV-Tat peptide.

The present inventors synthesized FITC and conducted a FACS experiment to verify its self-penetration efficacy using the cell-penetrating peptide RMAD1 derived from the human ADARB2 protein. As a result, it was found that the RMAD1 peptide penetrated cells much better than the HIV-Tat peptide.

In addition, the present inventors conducted an experiment by attaching an EGFP (Enhanced Green Fluorescence Protein) protein as a test cargo molecule to evaluate whether the RMAD1 peptide binds to the cargo molecule, penetrates well into the cells, and delivers the cargo molecule into the cells. A highly pure fusion protein was prepared through design and purification of the EGFP and RMAD1 fusion protein vector, and using this protein, various verification methods such as Western blotting, FACS, and confocal microscopy were used to identify that the EGFP-RMAD 1 fusion protein has a superior ability to deliver cargo molecules into cells compared to the HIV-Tat peptide.

The definitions of key terms used in the detailed description and claims of the present invention are as follows.

The term "cargo molecule" refers to a molecule other than a cargo molecule transduction domain or a fragment thereof that cannot inherently enter a target cell or cannot enter a target cell at a rate that is inherently useful, and refers to a target molecule itself before fusion with the cargo molecule transduction domain or a target molecular part of the cargo molecule transduction domain-target molecule complex. The cargo molecule refers to those selected from proteins including antibodies, peptides, polymers such as amino acids, nucleic acids, carbohydrates, and lipids, aptamers, liposomes, exosomes, and mixtures of one or more thereof.

The term "amino acid" and "amino acid residue" refers to a natural amino acid, non-natural amino acid, or modified amino acid. Unless otherwise indicated, all mentions about amino acids include general mentions about the amino acids and specific mentions about both D- and L-stereoisomers of the amino acids (so long as their structures allow such stereoisomeric forms) according to their names. Examples of the natural amino acids include alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr), and valine (Val). The non-natural amino acids include modified amino acid residues which are chemically modified, reversibly or irreversibly, or chemically blocked on their N-terminal amino group or their side chain groups, for example, N-methylated D and L amino acids or residues whose side chain functional groups are chemically modified to other functional groups.

The term "cargo molecule protein" refers to the case where a cargo molecule is a protein.

As a concept included in the "cargo molecule," the term "target protein" refers to a molecule other than a cargo molecule transduction domain or a fragment thereof that cannot inherently enter a target cell or cannot enter a target cell at a rate that is inherently useful, and refers to a target molecule itself before fusion with the cargo molecule transduction domain or a target molecular part of the cargo molecule transduction domain-target molecule complex. Target molecules include polypeptides, proteins, and peptides. Examples of target proteins belonging to target molecules include EGFP (Enhanced Green Fluorescent Protein), superoxide dismutase, epidermal growth factor, fibroblast growth factor, and catalase, but these are merely examples of some target proteins. It is obvious to those skilled in the art that the target protein is not limited thereto.

The term "recombinant cargo molecule" refers to a complex that includes a cargo molecule transduction domain and one or more cargo molecular parts, and is formed by genetic fusion or chemical bonding of the cargo molecule transduction domain and the cargo molecule. The term "fusion protein" refers to a recombinant cargo molecule formed by genetic fusion or chemical bonding with a cargo molecule protein and a cargo molecule transduction domain. As used herein, the term is used in the same sense as the recombinant cargo molecule protein.

In addition, the term "genetic fusion" refers to binding created by linear or covalent bonding through generic expression of DNA sequences encoding proteins. In addition, the term "target cell" refers to a cell into which a cargo molecules are delivered by the cargo molecule transduction domain, and the target cell refers to an *in vivo* or *ex vivo* cell. In other words, the target cell refers to *in vivo* cells, that is, the cells of the organ or tissue of living animals or humans, or microorganisms found in living animals or humans. In addition, the target cell is meant to include *ex vivo* cells, that is, cultured animal cells, human cells or microorganisms.

As used herein, the term "cargo molecule transduction domain" refers to a peptide that forms a covalent bond with cargo molecules such as high-molecular organic compounds, such as oligonucleotides, peptides, proteins, oligosaccharides, or polysaccharides, and allows the cargo molecules to be introduced into cells or tissues without the need for separate receptors, carriers, or energy. As used herein, the term "cargo molecule transduction domain" is used interchangeably with "protein transduction domain" or "cell penetrating domain."

In addition, as used herein, the expressions "penetration," "transduction," and "penetrate" are used interchangeably with respect to "introducing" cargo molecules such as proteins and peptides into cells or tissues.

As used herein, the term "conservative substitution" refers to modification of a cargo molecule transduction domain including substituting one or more amino acids by amino acids having similar biological or biochemical properties that do not cause loss of the biological or biochemical functions of the cargo molecule transduction domain.

As used herein, the term "conservative amino acid substitution" refers to a substitution to replace an amino acid residue by an amino acid residue having a similar side chain. Classes of the amino acid residue having a similar side chain are defined and well-known in the art. Such classes include amino acids with basic side chains (for example, lysine, arginine, histidine), amino acids with acidic side chains (for example, aspartic acid, glutamic acid), amino acids with uncharged polar side chains (for example, glycine, asparagine, glutamine, serine, threonine, tyrosine, cysteine), amino acids having non-polar side chains (for example, alanine, valine, leucine, isoleucine, proline, phenylalanine, methionine, tryptophan), amino acids having beta-branched side chains (for example, threonine, valine, isoleucine) and amino acids having aromatic side chains (for example, tyrosine, phenylalanine, tryptophan, and histidine).

Other terms used in the specification, claims, and drawings, unless specifically specified, are used in the sense generally used by those skilled in the art in the technical field to which the present invention pertains.

An embodiment of the present invention relates to a cargo molecule transduction domain that binds to cargo molecules and transports the cargo molecules into mammalian cells or tissues, wherein the cargo molecule transduction domain includes 1) a RMAD1 peptide consisting of SEQ ID NO: 1 derived from human ADARB2; or 2) a RMAD1 variant peptide consisting of 8 to 50 amino acids in which one or more amino acids are deleted, substituted, and/or added to the RMAD1 peptide.

In addition, an embodiment of the present invention relates to the cargo molecule transduction domain, wherein there is no limitation on an amino acid substitution in the RMAD1 variant peptide, but a conservative amino acid substitution is preferred.

In addition, an embodiment of the present invention relates to the cargo molecule transduction domain, wherein the RMAD1 variant peptide is a sequence in which the lysine residue position of SEQ ID NO: 1 is independently substituted with an arginine residue and/or the arginine residue position of SEQ ID NO: 1 is independently substituted with a lysine residue.

In addition, an embodiment of the present invention relates to the cargo molecule transduction domain, wherein in a peptide sequence in which one or more amino acids are deleted from the RMAD1 variant peptide, there is no particular limitation on the deleted amino acids, and preferably, one to six lysine residues and arginine residues among the amino acids of the RMAD1 peptide are deleted.

In addition, an embodiment of the present invention relates to a cargo molecule transduction domain, wherein a peptide sequence in which one or more amino acids are deleted and/or added to the RMAD1 peptide has amino acid deletions and/or additions in any one or more of the middle, N-terminus, and C-terminus of the sequence.

In addition, an embodiment of the present invention relates to the cargo molecule transduction domain, including: a RMAD1 peptide consisting of SEQ ID NO: 1 derived from human ADARB2; or a RMAD 1 variant peptide consisting of 8 to 50 amino acids in which one or more amino acids are deleted, substituted, and/or added to the RMAD1 peptide, and binding to cargo molecules and transporting the same into mammalian cells or tissues. The amino acid sequence of the RMAD1 peptide of SEQ ID NO: 1 is the same as "CKSKRRRRRRSKRKD." In addition, the amino acid mutation sequence among RMAD1 peptide variants refers to a peptide sequence in which amino acid mutation occurred individually at each amino acid residue position in SEQ ID NO: 1 above. In addition, the amino acid deleted sequence among the RMAD 1 peptide variants refers to a peptide sequence in which at least 1 to at most 7 amino acids are independently deleted among the amino acid sequences of SEQ ID NO: 1 above. Amino acid deletions may occur at either terminus or anywhere in the middle of a sequence, and consecutive or discontinuous amino acids may be deleted.

In addition, an embodiment of the present invention relates to a cargo molecule transduction domain, wherein the amino acid mutation sequence of the RMAD1 peptide variant is preferably a sequence in which the lysine residue position of SEQ ID NO: 1 is independently substituted with an arginine residue and/or the arginine residue position of SEQ ID NO: 1 is independently substituted with a lysine residue.

In addition, an embodiment of the present invention relates to a cargo molecule transduction domain, wherein, among the RMAD1 peptide variants, the sequence in which at least 1 to at most 7 amino acids are deleted is preferably a sequence in which 1 to 7 consecutive or non-consecutive lysine residues and arginine residues among the amino acids of the RMAD1 peptide are deleted.

In addition, the RMAD1 peptide variant of an embodiment of the present invention may be caused by overlapping amino acid substitutions and/or deletions and/or additions. For example, additional amino acid additions and deletions may occur in RMAD1 amino acid substitution variants. In addition, amino acid additions may occur in RMAD1 amino acid deletion variants. However, even through various combinations of amino acid substitutions and/or deletions and/or additions, there is no change in the ability to transport cargo molecules.

Specific examples of the cargo molecule transduction domain of an embodiment of the present invention include the peptides of SEQ ID NOs: 1 to 11.

In addition, an embodiment of the present invention relates to a cargo molecule transduction domain, wherein one or more selected from i) a RMAD1 peptide; or 2) a RMAD1 variant peptide consisting of 8 to 50 amino acids in which one or more amino acids are deleted, substituted, and/or added to the RMAD1 peptide is bound in the form of a dimer or higher-order multimer without a linker or through a linker. For example, the cargo molecule transduction domain of an embodiment of the present invention may include a sequence of i) a RMAD1 peptide, or 2) a RMAD1 variant peptide consisting of 8 to 50 amino acids in which one or more amino acids are deleted, substituted, and/or added to the RMAD1 peptide. In addition, a sequence in which the peptide of i) or ii) is repeated twice or more may be included, and a cargo molecule transduction domain in which the peptides of i) and ii) are linked may be included.

In addition, the linker is not particularly limited as long as it allows the cargo molecule transduction domain to maintain activity, but preferably includes an amino acid such as glycine, alanine, leucine, isoleucine, proline, serine, threonine, asparagine, aspartic acid, cysteine, glutamine, glutamic acid, lysine, arginine acid, and the like, to link each cargo molecule transduction domain monomer, more preferably several amino acids selected from valine, leucine, aspartic acid, glycine, alanine, proline, and the like, to link the same and most preferably 1 to 5 amino acids selected from glycine, valine, leucine, aspartic acid, and the like, in consideration of the ease of genetic manipulation. In addition to the above amino acid linkers and peptide linkers, chemical linkers may also be used as long as the activity of the cargo molecule transduction domain is maintained.

In addition, an embodiment of the present invention relates to a recombinant cargo molecule with improved cell membrane permeability in which a cargo molecule; and any one of the cargo molecule transduction domains are fused with one or more of an N-terminus and C-terminus of the cargo molecule.

The cargo molecule and the cargo molecule transduction domain may be fused without a linker or in the presence of a linker. In addition, the linker is not particularly limited as long as the cargo molecule transduction activity of the cargo molecule transduction domain and the activity of the cargo molecule are maintained, but preferably includes an amino acid such as glycine, alanine, leucine, isoleucine, proline, serine, threonine, asparagine, aspartic acid, cysteine, glutamine, glutamic acid, lysine, arginine acid, and the like, to link the cargo molecule transduction domain and the cargo molecule, more preferably several amino acids selected from valine, leucine, aspartic acid, glycine, alanine, proline, and the like, to link the same and most preferably 1 to 5 amino acids selected from glycine, valine, leucine, aspartic acid, and the like, in consideration of the ease of genetic manipulation. In addition to the above amino acid linkers and peptide linkers, chemical linkers may also be used as long as the activity of the cargo molecule transduction domain is maintained.

In addition, an embodiment of the present invention relates to a recombinant cargo molecule with improved cell membrane permeability, wherein the cargo molecule is a peptide, protein, or nucleic acid.

In addition, an embodiment of the present invention relates to a recombinant cargo molecule with improved cell membrane permeability, wherein the cargo molecule is a therapeutic protein, antigenic protein, or epitope peptide.

In addition, an embodiment of the present invention relates to a recombinant cargo molecule with improved cell membrane permeability, wherein the cargo molecule is an antioxidant protein.

In addition, an embodiment of the present invention relates to a drug for preventing or treating diseases containing the recombinant cargo molecule.

In addition, an embodiment of the present invention relates to cosmetics containing the recombinant cargo molecule. The cosmetics of an embodiment of the present invention may include color cosmetics such as foundation, lipstick, and eye shadow in addition to basic cosmetics such as lotion, cream, essence, oil-in-water or water-in-oil emulsion, and ointment.

In addition, an embodiment of the present invention relates to a medical device including the recombinant cargo molecule. The medical device of an embodiment of the present invention may include wound dressing, filler, and composite filler.

In addition, an embodiment of the present invention relates to a genetic construct containing a polynucleotide encoding the cargo molecule transduction domain.

Specific examples of the polynucleotide encoding the cargo molecule transduction domain of an embodiment of the present invention include the peptides of SEQ ID NOs: 12 to 22.

In addition, an embodiment of the present invention relates to an expression vector for expressing a recombinant cargo molecule protein with improved cell membrane permeability, wherein the vector includes the genetic construct.

In addition, an embodiment of the present invention relates to an expression vector for expressing a recombinant cargo molecule protein with improved cell membrane permeability, wherein the vector further includes a gene encoding a cargo molecule protein so that a recombinant cargo molecule protein in which a cargo molecule transduction domain and the cargo molecule protein are fused is able to be expressed.

In addition, an embodiment of the present invention relates to a recombinant cargo molecule in which the cargo molecule transduction domain which is fused with one or more of an N-terminus and a C-terminus of the cargo molecule for use in delivering cargo molecules into a cell in therapy..

In addition, an embodiment of the present invention relates to a recombinant cargo molecule for use in delivering cargo molecules into a cell in therapy. Proteins for disease prevention or treatment include growth factors such as epidermal growth factor, antibodies, antibody drugs, fusion proteins containing Fc of antibodies, antibody-drug complexes, protein drugs, enzymes, etc., but are not limited to these examples.

In addition, an embodiment of the present invention relates to a recombinant cargo molecule for use in delivering cargo molecules into a cell in therapy, wherein the cargo molecule is an antioxidant protein. It refers to antioxidant proteins such as superoxide dismutase and catalase, but is not limited to these examples.

In addition, an embodiment of the present invention relates to a cargo molecule transduction domain, wherein the cargo molecule transduction domain is one of SEQ ID NOs: 1 to 11.

In addition, an embodiment of the present invention relates to a cargo molecule transduction domain, wherein the genetic construct encoding the cargo molecule transduction domain is one of the polynucleotides of SEQ ID NOs: 12 to 22.

In addition, an embodiment of the present invention relates to a cargo molecule transduction domain, wherein the cargo molecule transduction domain includes polypeptide twice.

The cargo molecule transduction domain according to an embodiment of the present invention is interpreted to include a variant in which amino acid residues are conservatively substituted at a specific amino acid residue position of the RMAD1 peptide, or a peptide in which 1 to 5 amino acids are deleted from the N-terminus and/or C-terminus and/or the middle of the RMAD1 peptide or the variant thereof.

It is expected that the cargo molecule transduction domain of an embodiment of the present invention may still retain activity even when it has conservative amino acid substitutions.

In addition, the cargo molecule transduction domain variant according to an embodiment of the present invention has substantially the same function and/or effect as the cargo molecule transduction domain according to an embodiment of the present invention, and is interpreted to include cargo molecule transduction domain variants or fragments thereof having amino acid sequence homology of 80% or 85% or more, preferably 90% or more, and more preferably 95% or more.

In addition, an embodiment of the present invention is characterized in that the cargo molecule is selected from nucleic acids such as proteins, peptides, oligonucleotides, and polynucleotides, carbohydrates, lipids, and mixtures of one or more thereof.

In addition, an embodiment of the present invention is characterized in that the chemical bond between the cargo molecule transduction domain and the cargo molecule is a covalent bond or a non-covalent bond. The chemical bond may be a covalent bond or a non-covalent bond. Non-covalent bonds may include ionic bonds, bonds due to electrostatic attraction, or bonds due to hydrophobic interactions. In addition, the material that may bind to the cargo molecule transduction domain through ionic bonds or electrostatic attraction may be a charged material such as DNA or RNA.

In addition, an embodiment of the present invention relates to a recombinant cargo molecule that may easily penetrate into cells or tissues, wherein the cargo molecule transduction domain is one selected from SEQ ID NO: 1 to SEQ ID NO: 11. Representative peptides are shown in Table 1 for convenience of experiment.

In addition, an embodiment of the present invention relates to a polynucleotide, wherein the polynucleotide sequence encoding the cargo molecule transduction domain is one selected from SEQ ID NO: 12 to SEQ ID NO: 22. Representative polynucleotides are shown in Table 2 for convenience of experiment.

The pharmaceutical composition containing a recombinant cargo molecule, a polynucleotide encoding the same, or a vector including the polynucleotide as an active ingredient of an embodiment the present invention may be mixed with a carrier commonly accepted in the pharmaceutical field and formulated in various forms such as an external skin preparation, oral administration, spray, patch, or injection by conventional methods. Examples of oral compositions include tablets and gelatin capsules, and may contain diluents (example: lactose, dextrose, sucrose, mannitol, sorbitol, cellulose and/or glycine) and glydents (example: silica, talc, and stearic acid and its magnesium or calcium salts, and/or polyethylene glycol) in addition to the active ingredients. In addition, the tablets may preferably contain binding agents such as magnesium aluminum silicate, starch paste, gelatin, methyl cellulose, sodium carboxymethyl cellulose and/or polyvinyl pyrrolidine, and depending on the situation, may contain disintegration agents such as starch, agar, alginic acid or its sodium salt or similar mixture and/or absorbent, coloring, flavor and sweetener. Compositions for injection are preferably isotonic aqueous solutions or suspensions, and the compositions mentioned are sterile and/or contain auxiliaries (for example, preservatives, stabilizers, wetting or emulsifying agents, solution accelerators, salts and/or buffers for adjusting osmotic pressure). In addition, these compositions may contain other therapeutically useful substances.

The pharmaceutical preparation prepared as such may be administered orally or parenterally, that is, via intravenous, subcutaneous, intraperitoneal, or topical administration, according to the intended purposes. The daily dosage of 0.0001-100 mg/kg may be administered once a day or a few times a day. The dosage level for a specific patient may vary depending on the patient's weight, age, gender, health condition, administration time, administration method, excretion rate, and severity of disease.

The cargo molecule transduction domain newly discovered through an embodiment of the present invention has excellent cell penetration ability and is useful as a cargo molecule delivery material. Since this material is derived from the human body, it is safe as there is no risk of causing an immune response when administered to the human body.

In addition, the cargo molecule transduction domain of the present invention has a significantly superior cell penetration ability compared to other conventional cargo molecule transduction domains, enabling smooth penetration of various substances that are difficult to penetrate cells, such as protein drugs and antigen epitopes, and can be applied as drugs, cosmetics, etc. do.

### BRIEF DESCRIPTION OF THE DRAWINGS

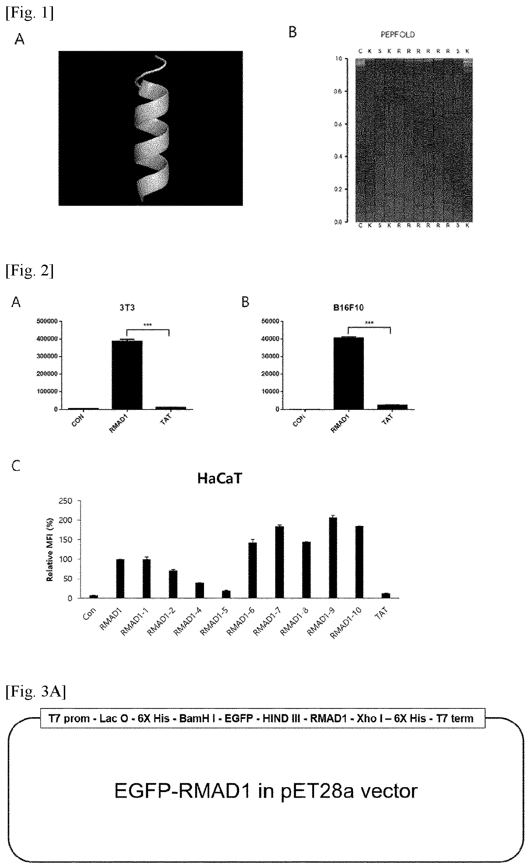

FIG. 1 shows a secondary structure prediction diagram of a RMAD1 cell-penetrating peptide. FIG. 1A shows a secondary structure of the peptide predicted using the Pep-fold3 program. FIG. 1B is a graph showing the Pepfold of the peptide.
FIGS. 2A and B show the results of measuring the amount (fluorescence value) of FITC RMAD1 penetrated into cells after treating RMAD1 conjugated with 2.5 uM of FITC and TAT conjugated with 2.5 uM of FITC using two cell lines of 3T3 and B16F10. FIG. 2C shows the result of measuring the amount (fluorescence value) of FITC RMAD1 and its variant penetrated into cells after conjugating RMAD1 and its variants with FITC and treating the same with 2.5 uM. In addition to the untreated control group, one treated with 2.5 uM of FITC TAT was used as a control group.

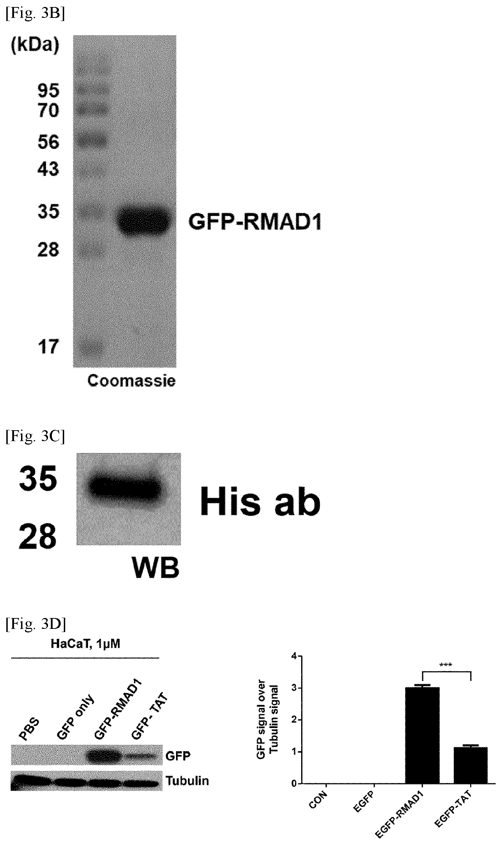

FIG. 3 shows the vector map of RMAD1 fused with EGFP and the purity of the purified protein. FIG. 3A shows which restriction enzyme is used to sub-clone EGFP-RMAD1 into the pET28a vector. FIG. 3B shows the results of identifying the molecular weight of purified EGFP-RMAD1 using Coomassie blue. FIG. 3C shows a Western blot result of identifying the molecular weight of purified EGFP-RMAD1 and whether the purification was successful. FIG. 3D shows the results of comparing and identifying the delivery rate of EGFP with that of EGFP and EGFP-TAT using Western blotting after treating purified EGFP-RMAD1, EGFP, and EGFP-TAT in HaCaT cells.

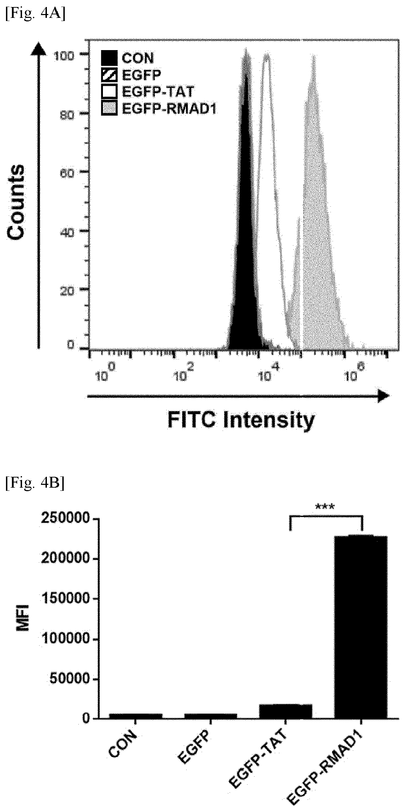

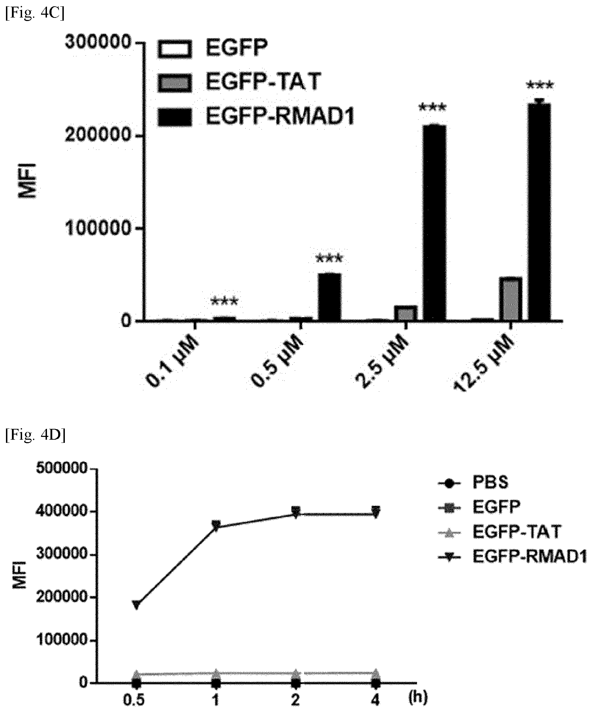

FIG. 4 shows the results of identifying cell permeability using FACS. FIG. 4A shows the results of identifying the amount of EGFP delivered to the cells through FACS after treating HaCaT cells with 2.5 uM of EGFP-RMAD1, EGFP, and EGFP-TAT. FIG. 4B is the result of showing the fluorescence values from FIG. 4A as a histogram. FIG. 4C shows the results of cell permeability of EGFP-RMAD1, EGFP, and EGFP-TAT identified by FACS after treatment at each dose for 2 hours. FIG. 4D shows the results of cell permeability of EGFP-RMAD1, EGFP, and EGFP-TAT identified by FACS after treatment with 2.5 uM per time.

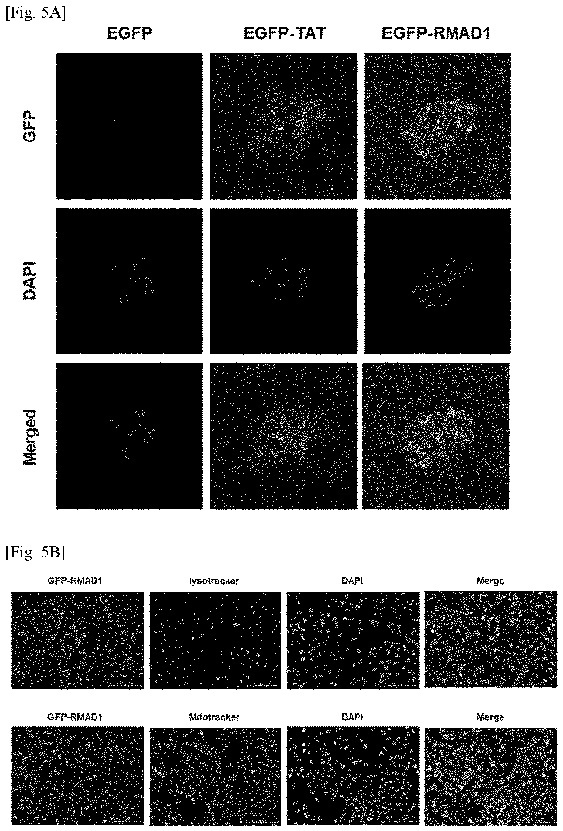

FIG. 5A shows the results of identifying cell permeability through confocal microscopy of the image of EGFP delivered to the cells after treating HaCaT cells with 2.5 uM of EGFP-RMAD1, EGFP, and EGFP-TAT. FIG. 5B shows the results of identifying the position of the fusion protein with a fluorescence microscope using a lysotracker or mitotracker after treating the fusion protein in the same manner as in FIG. 5A.

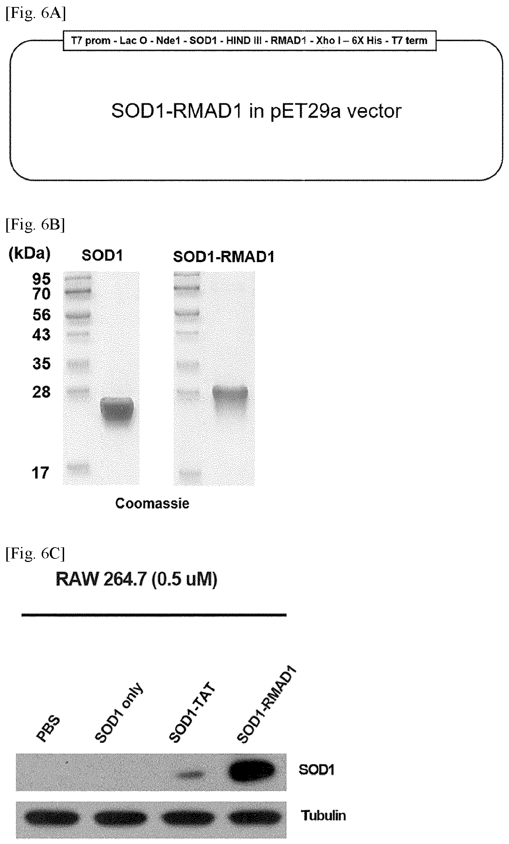

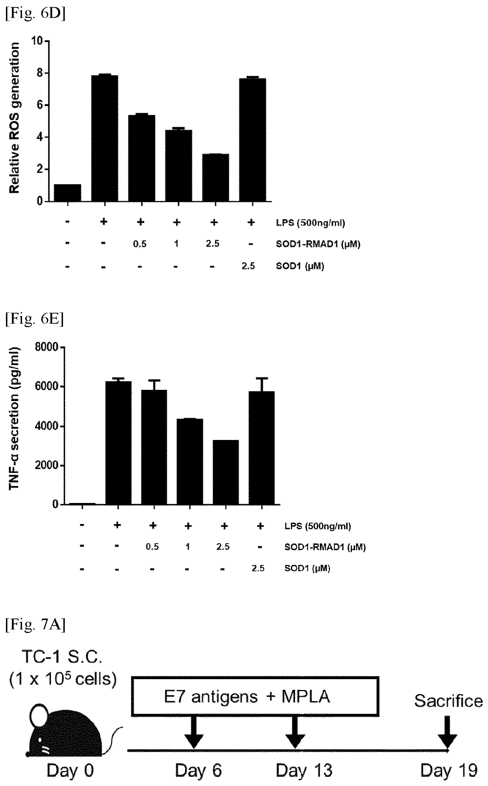

FIG. 6 shows the vector map of RMAD1 fused with SOD1 and the purity of the purified protein. FIG. 6A shows which restriction enzyme is used to sub-clone SOD1-RMAD1 into the pET28a vector. FIG. 6B shows the results of identifying the molecular weight of purified SOD1-RMAD1 using Coomassie blue. FIG. 6C shows a Western blot result of identifying the molecular weight of purified SOD1-RMAD1 and whether the purification was successful. FIG. 6D shows the results of comparing and identifying the relative production rate of reactive oxygen species using Western blotting after treating purified SOD1-RMAD1, SOD1, and SOD1-TAT in HaCaT cells treated with 500 ng/ml of LPS. FIG. 6E shows the result of detecting TNF-α obtained by culturing the cells in serum-free medium for 1 hour and then treating the same in each well with 0.5, 1, and 2.5 uM of SOD1-RMAD1 for 2 hours, and then treating the same in each well with 500 ng/ml of LPS for 16 hours, and then recovering the medium and centrifuging the same at 1,000 g for 10 minutes.

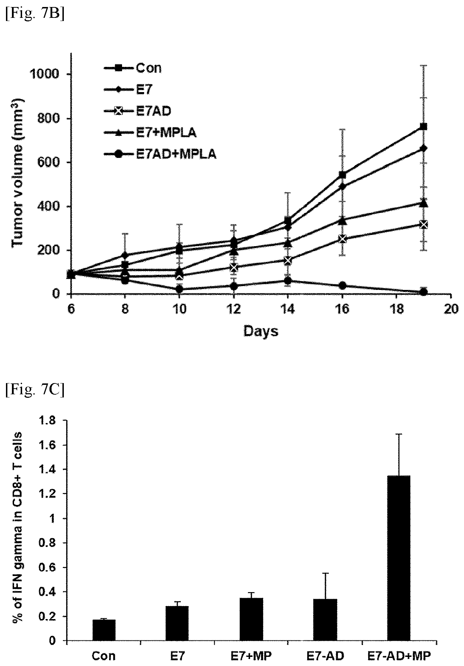

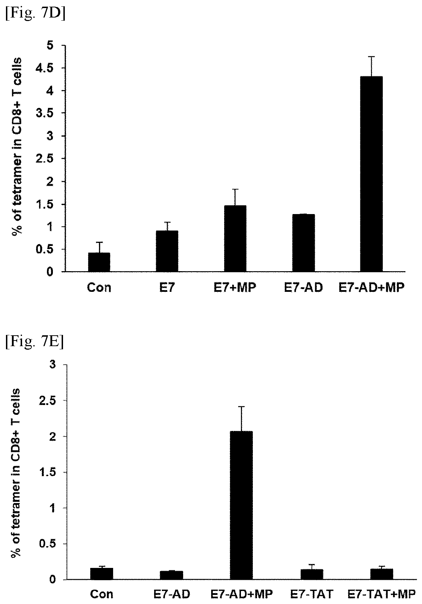

FIG. 7 shows experiments and experimental results identifying anticancer vaccine efficacy by fusing RMAD-1 with a peptide antigen. FIG. 7A shows the experimental procedure in which mice were subcutaneously injected with TC-1 cells, injected with E7 antigen and MPLA on days 6 and 13, respectively, and sacrificed on day 19. FIG. 7B a graph showing the change in tumor size according to the control group (Con), E7 antigen administration group (E7), E7-RMAD1 fusion peptide treatment group (E7AD), E7 antigen + MPLA (immune adjuvant and TLR4 agonist) treatment group (E7+MPLA), and E7-RMAD1 fusion peptide + MPLA treatment group (E7AD+MPLA). In FIGS. 7C and 7D, as a result of analyzing immune cells in the mouse spleen, it was identified that the number of antigen-specific CD8+ T cells increased in the E7AD including RMAD1 and E7AD + MPLA treatment groups, and the activation of CD8+ T cells was identified through increased expression of IFN-γ. In FIG. 7E, as a result of comparing and analyzing E7-TAT bound to TAT, a conventional cargo molecule transduction domain, using blood immune cells, it was shown that antigen-specific CD8 ⁺ T cells are effectively increased in the E7-RMAD1 treatment group.

### DESCRIPTION OF EXEMPLARY EMBODIMENTS

Hereinafter, the configuration of the present invention will be described in more detail through specific examples. However, it is obvious to those skilled in the technical field to which the present invention pertains that the scope of the present invention is not limited to the description of the examples, but is defined by the claims.

### 1. Cell Culture

HaCaT and RAW264.7 cells were cultured in cell culture medium supplemented with 10% of fetal bovine serum (FBS) and antibiotic solution (100 units/ml of penicillin, 100 µg/ml of streptomycin) in DMEM medium. TC-1 cells were cultured in a cell culture medium supplemented with 10% of fetal bovine serum (FBS) and antibiotic solution (100 units/ml of penicillin, 100 µg/ml of streptomycin) G418 (0.4 mg/ml) in RPMI medium. Cells were cultured under conditions maintained at 37°C, 95% humidity, and 5% CO₂, and when the cells were 70-80% adhered to the culture dish, they were treated with trypsin-EDTA and subcultured. HaCaT cells were distributed and cultured from Professor Kim Tae Yoon of the College of Medicine, The Catholic University of Korea, 3T3 and B16F10 cells were distributed and cultured from the Seoul National University Cell Line Bank, and RAW264.7 cells and TC-1 were distributed and cultured from the laboratory of Professor Kim Sang-Beom, College of Pharmacy, Sahmyook University.

### 2. RMAD1 Construction

The following oligonucleotide was used and annealed at 94°C for 5 minutes and then at 25°C for 1 hour.
Sense oligonucleotides:
   5'-agctttgcaagtccaagaggaggaggaggcggaggtccaagcggaaagatc-3' (SEQ ID NO: 23)
Antisense oligonucleotides:
   5'-tcgagatctttccgcttggacctccgcctcctcctcctcttggacttgcaa-3' (SEQ ID NO: 24)

Thereafter, the HindIII and XhoI sites of the pET-28a vector were cleaved, and the RMAD1 coding oligonucleotide pair prepared above was linked. The recombinant RMAD1 was transformed into *Escherichia coli* strain DH5α (RH618). The DNA isolated from the transformed cells was cleaved at the BamHI and HindIII sites and linked to the EGFP (Enhanced Green Fluorescence Protein) gene.

### 3. Protein Purification

EGFP-RMAD1, EGFP, and EGFP-TAT proteins were purified using the pET28a plasmid vector containing N- and C-terminal 6X his tags. BL21-Codon Plus cells were transformed, and colonies were inoculated into medium and grown. Large-scale cells were cultured in LB medium until OD 600 reached 0.5, and protein expression was induced using 0.5 mM of IPTG for 16 hours at 4°C. Cell pellets were obtained from centrifugation and disrupted by sonication in 50 mM Tris buffer pH 7.5 containing 300 mM NaCl. Thereafter, the supernatant was obtained by centrifugation at 20,000 g for 30 minutes. This was poured onto a Ni-NTA resin column. Washing stages were performed with 50mM Tris buffer, pH 7.5, containing 300 mM NaCl, 5% glycerol, and 15mM imidazole. Proteins were isolated from the column using 10 ml of elution buffer (50 mM Tris pH 7.5, 300 mM NaCl, 5% of glycerol, and 300 mM of imidazole), and then endotoxin was removed using a Mustang column (Pall, MSTG25E3). From LAL (limulus amebocyte lysate) analysis, an appropriate protein of 0.5 EU/mg or less was used in the entire experiment.

### 4. Cell Introduction of EGFP-RMAD1 Fusion Protein using FACS

In order to evaluate the cell permeability of RMAD1 in HaCaT skin cells, 1 x 10⁵ of HaCaT cells were attached to a 24-well plate 12 hours in advance. Thereafter, the cells were washed using serum-free DMEM medium and then treated with 2.5 uM of protein in serum-free medium for 2 hours. After 2 hours, the cells were washed a total of 3 times with serum-free medium, and then the cells were detached from the plate by treatment with 150 ul of trypsin, and then neutralized by adding 850 ul of serum-containing medium. After centrifugation at 500 g for 10 minutes, the medium was removed, and proteins on the cells were removed using 500 ul of FACS buffer (1% BSA, 0.1% sodium azide). After repeating the wash twice, FACS analysis was performed by adding FACS buffer.

### 5. Cell Introduction of EGFP-RMAD1 Fusion Protein using Confocal and Fluorescence Microscopy

In order to evaluate the cell permeability of RMAD1 in HaCaT skin cells, a 9 mm coverslip was placed on a 24-well plate 12 hours in advance, and then 1 x 10⁵ of HaCaT cells were attached. Thereafter, the cells were washed using serum-free DMEM medium and then treated with 2.5 uM of protein in serum-free medium for 2 hours. After 2 hours, the cells were washed a total of 3 times with serum-free medium, and then hoechst was diluted in PBS at a ratio of 1:1000 and stained for 5 minutes. Thereafter, the wells were washed a total of five times using PBS, and the glass cover slips were separated from the wells to remove moisture and proceed with mounting. Thereafter, images were obtained using a confocal microscope and fluorescence expression was identified. In the case of fluorescence microscopy, 1 x 10⁴ of HaCaT cells were attached to a 96-well plate the day before, and then protein was treated in the same manner as above. Then, 30 minutes before the end of the experiment, it was washed with serum-free medium and treated with Mitotracker-deep red FM (1:2000) or Lysotracer Red DND-99 (1:2000), and 5 minutes before the end of the experiment, hoechst was diluted in PBS at a ratio of 1:1000 and stained for 5 minutes. Thereafter, the fluorescence signal was observed using BioTek's Lionheart FX automated microscope equipment.

### 6. Western Blotting

In order to evaluate the cell permeability of RMAD1 in HaCaT skin cells, 3 x 10⁵ HaCaT cells were attached to a 12-well plate 12 hours in advance. Thereafter, the cells were washed using serum-free DMEM medium and then treated with 1 uM of protein in serum-free medium for 2 hours. After 2 hours, the cells were washed a total of 3 times using serum-free medium to remove any proteins that may have remained on the cell surface. Thereafter, the protein in the supernatant was quantified through RIPAlysis buffer and centrifugation, and then 30 ug of protein was mixed with 5X sample buffer. The prepared protein sample was boiled for 10 minutes and separated according to molecular weight using a 12% of SDS-PAGE gel. After electrophoresis was completed, the protein was transferred to a PVDF membrane and blocked for 1 hour using TBS-T buffer containing 5% of skim milk. Then, in order to measure protein expression, reaction was performed using a GFP antibody as the primary antibody, and then using a horseradish peroxidase-conjugated anti-rabbit antibody as the secondary antibody. After washing with TBS-T buffer, the cell permeability of each protein was measured.

### 7. Evaluation of Improvement in Antioxidant Efficacy using SOD1-RMAD1 Fusion Protein

In order to evaluate the antioxidant efficacy of RMAD1 in RAW 264.7 macrophages, 5 x 10⁴ of RAW264.7 cells were attached to a 24-well plate 12 hours in advance. Thereafter, the cells were cultured in serum-free medium for 1 hour and then treated with 0.5, 1, and 2.5 uM of SOD1-RMAD1 in each well for 2 hours. Next, the cells in each well were treated with 500 ng/ml of LPS (lipopolysaccharide) for 16 hours. Thereafter, the cells were washed a total of three times with serum-free medium, treated with 150 ul of trypsin to drop the cells, and then neutralized by adding 850 ul of serum-containing medium. After centrifugation at 500 X g for 10 minutes, the medium was removed, and then washed with 500 ul of HBSS. Thereafter, staining was performed for 10 minutes using CM-H2DCFDA (Thermo, C6827), and then washed twice with FACS buffer and then subjected to FACS analysis.

### 8. Evaluation of SOD1-RMAD1 TNF-α Inhibition through SOD1-RMAD1 Antioxidant

In order to evaluate the anti-inflammatory efficacy of RAMD1 in RAW 264.7 macrophages, 5 x 10⁴ of RAW264.7 cells were attached to a 24-well plate 12 hours in advance. Thereafter, the cells were cultured in serum-free medium for 1 hour and then treated with 0.5, 1, and 2.5 uM in each well for 2 hours. Next, each well was treated with 500 ng/ml of LPS for 16 hours. Thereafter, the medium was recovered, centrifuged at 1,000 X g for 10 minutes, and TNF was detected.

### 9. Syngeneic Mouse Tumor Model and Tumor Measurements

TC-1 cells were maintained in RPMI supplemented with serum and G418, and the cells were dropped with trypsin, then neutralized and washed. Thereafter, 1 x 10⁵ cells were subcutaneously injected into the right side of 6-week-old C57BL/6 mice, and on days 6 and 13, E7, E7-AD, and E7-TAT were subcutaneously injected at 8.8 nmol into the left side of the mouse, and an additional 25 ug was mixed and injected into the MPLA group. Measurements were made with digital calipers and calculations were made according to (0.52 X length X width²). Mice were euthanized when the tumor size became 1,000 mm³ or more.

### 10. Spleen and Blood Immune Cell Analysis

After TC-1 was injected into C57BL/6 mice, each antigen and MPLA (Monophosphoryl-Lipid A) were injected according to the above schedule, and the mice were sacrificed on the 19th day. Thereafter, spleen cells were dissociated using RPMI medium containing 2% of FBS and 1% of streptomycin. Red blood cells were lysed from the spleen and blood cells using red blood cell lysis solution (BD, 555899), and each cell was stained with CD8, CD3, IFN-γ, and E7 (H-2D^{b}-HPV16) tetramer antibodies for 30 minutes at 4°C. For intracellular staining, a fixation/permeabilization solution kit (BD, 555028) was used. In this connection, spleen cells were restimulated by treating the same with E7₄₉₋₅₇ (RAHYNIVTF) peptide for 16 hours, and then intracellular IFN-γ of CD8⁺ T cells was detected through FACS.

### Result 1: RMAD1 Peptide Secondary Structure Prediction Diagram

In order to predict the peptide structure of RMAD1, the PEP-FOLD3 De novo peptide structure prediction program was used. By entering the amino acid sequence of RMAD1, the model predicted by the program was imaged as a two-dimensional predicted structure using the PyMOL 2.4 program [FIG. 1A]. In the prediction profile used for secondary structure prediction analysis, red represents a spiral, green represents an extension, and blue represents a coil, from which a secondary prediction model was derived [FIG. 1B].

### Result 2: RMAD1-FITC Synthetic Peptide Cell Penetration Efficiency

Using a synthetic peptide attached with fluorescent FITC, the cell penetration efficiency of the fluorescent protein FITC bound to RMAD1 and its variants was identified using cell lines 3T3, B16F10, and HACAT. In the case of cells to which FITC was not attached, it was difficult to identify the permeability of the peptide, so fluorescent FITC was attached to identify cell permeability. Each cell line was treated with FITC-attached peptides of RMAD-FITC and TAT-FITC peptides at the same concentration of 2.5 uM for 2 hours. As a result, the RMAD1-FITC synthetic peptide showed a significantly higher cell penetration level than the control TAT-FITC peptide in both cell lines [FIGS. 2A and 2B]. In addition, it was identified that better permeation efficacy than TAT was maintained in HACAT cells even through amino acid substitution, removal, and addition [FIG. 2C].

### Result 3: Schematic Diagram and Purification of EGFP-RMAD1 Fusion Protein

In order to produce a fusion protein containing a human-derived cell-penetrating peptide and EGFP, the sequence encoding the RMAD1 polypeptide (tgc aag tcc aag agg agg agg agg cgg agg tcc aag cgg aaa gat; SEQ ID NO: 12) was cloned into Hind III and Xho I sites of pET-28a plasmid. RMAD1 coding DNA and EGFP cDNA were recombined, BamH I and Hind III sites were cleaved, and EGFP-RMAD1 was cloned into pET-28a plasmid [FIG. 3A]. EGFP and EGFP-TAT to be used as a control group were also cloned into the same vector, pET-28a plasmid, in the same manner as above. In the case of the fusion protein containing his-tag, after binding to the Ni-NTA column, it was washed with a low concentration of 15 mM of imidazole solution and then eluted with 300 mM of imidazole solution. In the case of the eluted fusion protein product, a total of 10 ug of protein was loaded using SDS-PAGE, and the molecular weight of the purified fusion protein was identified using coomassie blue staining reagent [FIG. 3B]. In addition, Western blotting was performed to identify the expression of the EGFP-RMAD1 protein containing the his-tag in the stained fusion protein, and through his-antibody, it was identified that the molecular weight of the fusion protein containing the his-tag was consistent with the coomassie blue staining results [FIGS. 3B and 3C].

### Result 4: Introduction of RMAD1 Fusion Protein into HaCaT Cells

In order to identify the cell penetration efficiency of the RMAD1 fusion protein, the fusion protein was introduced into HaCaT, a human skin cell line, and the efficiency was identified. Recombinant proteins EGFP, EGFP-TAT, and EGFP-RMAD1 were treated at the same concentration of 1 uM to HaCaT cells for 2 hours. As a result, it was identified that the EGFP-RMAD1 fusion protein showed a significantly higher level of cell penetration, and secondly, the EGFP-TAT fusion protein containing TAT, which is well known as a cell-penetrating peptide, showed a cell permeability about 60% lower than that of EGFP-RMAD1. In addition, it was identified that the EGFP protein itself did not penetrate into cells [FIG. 3D].

The cellular efficacy of the RMAD1 fusion protein was analyzed using different experimental techniques, and intracellular fluorescence was measured using a flow cytometer to identify the efficiency according to concentration and over time. Specifically, in order to analyze the introduction efficiency according to concentration, a total of 4 concentrations of fusion proteins were treated for 2 hours at a 5-fold ratio from 0.1 uM to 12.5 uM concentration. As a result, it was identified that the RMAD1 fusion protein was delivered to HaCaT cells in a concentration-dependent manner, and similar to the Western blotting results above, a significantly higher cell permeability was identified compared to the TAT fusion protein [FIGS. 4A, 4B, and 4C]. In addition, in order to analyze the introduction efficiency according to the treatment time, a fusion protein at a concentration of 2.5 uM was cultured for 30 minutes to 4 hours and FACS analysis was performed. As a result, it was identified that the RMAD1 fusion protein was delivered to HaCaT cells in a time-dependent manner, and the amount of protein penetrating steadily increased for up to 2 hours. In addition, it was identified once again that it delivered proteins more efficiently than TAT, a well-known cell-penetrating domain [FIG. 4D].

The position of the EGFP-RMAD1 fusion protein introduced into a cell was checked to identify whether it was actually introduced into the cell. In addition, in order to identify the cell penetration efficacy once again, HaCaT cells were treated with the EGFP-RMAD1 fusion protein and then were observed using fluorescence microscopy and confocal microscopy. As a result, in the case of the control EGFP protein, no fluorescence signal was observed, but in the case of EGFP-TAT and EGFP-RMAD1, it was identified that they were mainly detected in the cytoplasm as a result of confocal microscopy [FIG. 5A]. In addition, in the case of EGFP-RMAD1, a significantly higher fluorescence signal was observed compared to the EGFP-TAT fusion protein, which was consistent with the Western blotting and FACS results above. The same result was obtained in fluorescence microscopy, and it was observed that most of the introduced EGFP-RMAD1 was not in the same position as the lysotracker and mitotracker, which indicate the position of lysosomes or mitochondria. This reaffirmed that the introduced fusion protein was located in the cytoplasm. [FIG. 5B].

### Result 5: Schematic Diagram and Purification of SOD1-RMAD1 Fusion Protein

In order to produce a fusion protein containing a human-derived cell-penetrating peptide and SOD1, the sequence encoding the RMAD1 polypeptide (tgc aag tcc aag agg agg agg agg cgg agg tcc aag cgg aaa gat; SEQ ID NO: 12) was cloned into Hind III and Xho I sites of pET-28a plasmid. RMAD1 coding DNA and SOD1 cDNA were recombined, Nde I and Hind III sites were cleaved, and SOD1-RMAD1 was cloned into pET-29a plasmid [FIG. 6A]. SOD1 to be used as a control group was also cloned into the same vector, pET-29a plasmid, in the same manner as above. In the case of the fusion protein containing his-tag, after binding to the Ni-NTA column, it was washed with a low concentration of 15 mM of imidazole solution and then eluted with 300 mM of imidazole solution. In the case of the eluted fusion protein product, a total of 10 ug of protein was loaded using SDS-PAGE, and the molecular weight of the purified fusion protein was identified using coomassie blue staining reagent [FIG. 6B].

### Result 6: Introduction of Fusion Protein into RAW264.7 Cells and Evaluation of Improvement in Antioxidant Efficacy

SOD1, which is widely known as an antioxidant enzyme, was fused with a cell-penetrating peptide, and the cell-penetrating ability and activity maintenance were evaluated using RAW264.7 cells. The recombinant proteins SOD1, SOD1-TAT, and SOD1-RMAD1 were treated at the same concentration of 0.5 uM to RAW264.7 cells for 2 hours. As a result, it was identified that the SOD1-RMAD1 fusion protein showed a significantly higher level of cell penetration. It was identified that the SOD1-TAT fusion protein containing TAT, a conventionally well-known cell-penetrating peptide, showed a lower level of cell penetration than SOD1-RMAD1, and that SOD1 itself did not penetrate cells [FIG. 6C].

Antioxidant and anti-inflammatory efficacies were evaluated to identify that the fusion protein introduced into cells was actually active within the cells. In order to evaluate antioxidant efficacy, the ability to remove reactive oxygen species (ROS) was identified. Specifically, RAW264.7 cells were treated with the SOD1-RMAD1 fusion protein at concentrations of 0.5, 1, and 2.5 uM for 2 hours, and then treated with LPS, which induces reactive oxygen species, for a total of 16 hours. Thereafter, as a result of evaluating the amount of reactive oxygen species through FACS analysis using CM-H2DCFDA, it was identified that the SOD1-RMAD1 fusion protein effectively inhibited intracellular reactive oxygen species production [FIG. 6D]. In addition, in order to evaluate the anti-inflammatory efficacy, the fusion protein and LPS were treated as in the antioxidant efficacy experiment above. Thereafter, it was identified that the fusion protein effectively improved the anti-inflammatory efficacy compared to SOD1 through the detection of TNF-α, an inflammatory factor, in the medium [FIG. 6E].

### Result 7: Evaluation of Anticancer Vaccine Efficacy using Peptide Antigen and RMAD1 Fusion Peptide

By quickly increasing intracellular permeability, it was expected to prevent peptide antigen degradation *in vivo* and effectively create antigen-specific T cells by recognizing the antigen in innate immune cells. In this regard, anticancer vaccine efficacy was identified by fusing RMAD-1 with peptide antigen. Antigen-specific T cells were evaluated using TC-1 cells overexpressing HPV-16/18-derived E7 and E7 peptide bound to RMAD1. In addition, MPLA (Monophosphoryl lipid A), a well-known immune adjuvant and TLR4 agonist, was used to identify a decrease in tumor size. As a result, E7AD was identified to have a higher anticancer ability compared to E7 alone, and a significant tumor reduction was identified also in the E7AD + MPLA group to which MPLA was added compared to E7 + MPLA [FIGS. 7A and 7B]. In addition, as a result of analyzing immune cells in the spleen, it was identified that the number of antigen-specific CD8⁺ T cells increased in the E7AD and E7AD + MPLA groups containing RMAD1. In addition, the activation of CD8⁺ T cells was identified through identification of increased expression of IFN-γ [FIGS. 7C and 7D]. Lastly, as a result of comparison with the conventional cell-penetrating peptide of E7-TAT using blood immune cells, it was identified that the group containing RMAD1 effectively increased antigen-specific CD8 ⁺ T cells [FIG. 7E].

Table 1 below shows the amino acid sequences of 11 specific examples of the cargo molecule transduction domains of an embodiment of the present invention, which are respectively SEQ ID NO: 1 to SEQ ID NO: 11 in the order of the table.

Table 2 below shows the polynucleotide sequences encoding 11 specific examples of the cargo molecule transduction domains of an embodiment of the present invention, which are respectively SEQ ID NO: 12 to SEQ ID NO: 22 in the order of the table.

**[Table 1]**

| Name | amino acid sequence | characteristics | Seq.ID |
|---|---|---|---|
| RMAD1 | CKSKRRRRRRSKRKD | - | 1 |
| RMAD1-1 | CRSKRRRRRRSRRRD | K12R, K14R substitution | 2 |
| RMAD1-2 | CKSKKKKKKKSKRKD | 6R of 5 to 10 > substituted with 6K of 5 to 10 | 3 |
| RMAD1-3 | CKSKRRRRRRS | C-terminal KRKD deletion | 4 |
| RMAD1-4 | CKSKRRRRSKR | RR deletions among 5 to 10, C-terminal KD deletions | 5 |
| RMAD1-5 | SKRRRRSKRKD | N-terminal CK deletions, RR deletions among 5 to 10 | 6 |
| RMAD1-6 | YKSKRRRRRRSKRKD | C1Y substitution | 7 |
| RMAD1-7 | KCKSKRRRRRRSKRKDKV | N-terminal K addition, C-terminal KV addition | 8 |
| RMAD1-8 | KCKSKRRRRRRSKRKDKVS | N-terminal K addition, C-terminal KVS addition | 9 |
| RMAD1-9 | LKCKSKRRRRRRSKRKDKV | N-terminal LK addition, C-terminal KV addition | 10 |
| RMAD1-10 | LKCKSKRRRRRRSKRKDKV S | N-terminal LK addition, C-terminal KVS addition | 11 |

**[Table 2]**

| Name | polynucleotide sequence | Seq.ID |
|---|---|---|
| RMAD1 | tgc aag tcc aag agg agg agg agg cgg agg tcc aag cgg aaa gat | 12 |
| RMAD1-1 | tgc agg tcc aag agg agg agg agg cgg agg tcc agg cgg agg gat | 13 |
| RMAD1-2 | tgc aag tcc aag aag aag aag aag aag aag tcc aag cgg aaa gat | 14 |
| RMAD1-3 | tgc aag tcc aag agg agg agg agg cgg agg tcc | 15 |
| RMAD1-4 | tgc aag tcc aag agg agg cgg agg tcc aag cgg | 16 |
| RMAD1-5 | tcc aag agg agg cgg agg tcc aag cgg aaa gat | 17 |
| RMAD1-6 | tat aag tcc aag agg agg agg agg cgg agg tcc aag cgg aaa gat | 18 |
| RMAD1-7 | aaa tgc aag tcc aag agg agg agg agg cgg agg tcc aag cgg aaa gat aaa gta | 19 |
| RMAD1-8 | aaa tgc aag tcc aag agg agg agg agg cgg agg tcc aag cgg aaa gat aaa gta agc | 20 |
| RMAD1-9 | ctc aaa tgc aag tcc aag agg agg agg agg cgg agg tcc aag cgg aaa gat aaa gta | 21 |
| RMAD1-10 | ctc aaa tgc aag tcc aag agg agg agg agg cgg agg tcc aag cgg aaa gat aaa gta agc | 22 |

## Claims

1. A cargo molecule transduction domain that binds to cargo molecules and transports the cargo molecules into mammalian cells or tissues, the cargo molecule transduction domain comprising:
a RMAD1 peptide selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, and SEQ ID NO: 11 derived from human ADARB2.

2. The cargo molecule transduction domain according to claim 1, wherein one or more of a RMAD1 peptide selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, and SEQ ID NO: 11 derived from human ADARB2 is bound in the form of a dimer or higher-order multimer without a linker or through a linker.

3. A recombinant cargo molecule with improved cell membrane permeability in which a cargo molecule; and any one of the cargo molecule transduction domains selected from claim 1 or 2 are fused with one or more of an N-terminus and C-terminus of the cargo molecule.

4. The recombinant cargo molecule of claim 3, wherein the cargo molecule is a peptide, protein, or nucleic acid.

5. The recombinant cargo molecule of claim 3, wherein the cargo molecule is a therapeutic protein, an antigenic protein, or an epitope peptide.

6. The recombinant cargo molecule of claim 3, wherein the cargo molecule is an antioxidant protein.

7. A drug containing the recombinant cargo molecule of any one of claims 3 to 6.

8. Cosmetics containing the recombinant cargo molecule of any one of claims 3 to 6.

9. A genetic construct containing a polynucleotide encoding the cargo molecule transduction domain of claim 1 or 2.

10. An expression vector for expressing a recombinant cargo molecule protein with improved cell membrane permeability, the expression vector comprising the genetic construct of claim 9.

11. The expression vector of claim 10, further comprising a gene encoding a cargo molecule protein so that a recombinant cargo molecule protein in which a cargo molecule transduction domain and the cargo molecule protein are fused is able to be expressed.

12. A recombinant cargo molecule in which the cargo molecule transduction domain of claim 1 or 2 is fused with one or more of an N-terminus and a C-terminus of the cargo molecule for use in therapy.

13. The recombinant cargo molecule for use as in claim 12, wherein the cargo molecule is a therapeutic protein.

14. The recombinant cargo molecule for use as in claim 12, wherein the cargo molecule is an antioxidant protein.

## Patentansprüche

1. Cargomolekül-Transduktionsdomäne, die an Cargomoleküle bindet und die Cargomoleküle in Säugetierzellen oder -gewebe transportiert, die Cargomolekül-Transduktionsdomäne umfassend:
ein RMAD1-Peptid, ausgewählt aus der Gruppe, bestehend aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 und SEQ ID NO: 11 abgeleitet von menschlichem ADARB2.

2. Cargomolekül-Transduktionsdomäne gemäß Anspruch 1, wobei eines oder mehrere von einem RMAD1-Peptid, das aus der Gruppe ausgewählt ist, bestehend aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 und SEQ ID NO: 11 abgeleitet von menschlichem ADARB2 in der Form eines Dimers oder Multimers höherer Ordnung ohne einen Linker oder über einen Linker gebunden ist.

3. Rekombinantes Cargomolekül mit verbesserter Zellmembranpermeabilität, bei dem ein Cargomolekül; und eine beliebige der Cargomolekül-Transduktionsdomänen, ausgewählt aus Anspruch 1 oder 2, mit einem oder mehreren von einem N-Terminus und C-Terminus des Cargomoleküls fusioniert sind.

4. Rekombinantes Cargomolekül gemäß Anspruch 3, wobei das Cargomolekül ein Peptid, Protein oder eine Nukleinsäure ist.

5. Rekombinantes Cargomolekül gemäß Anspruch 3, wobei das Cargomolekül ein therapeutisches Protein, ein antigenes Protein oder ein Epitoppeptid ist.

6. Rekombinantes Cargomolekül gemäß Anspruch 3, wobei das Cargomolekül ein antioxidatives Protein ist.

7. Arzneimittel, das rekombinante Cargomolekül nach einem der Ansprüche 3 bis 6 enthaltend.

8. Kosmetika, das rekombinante Cargomolekül nach einem der Ansprüche 3 bis 6 enthaltend.

9. Genetisches Konstrukt, ein Polynukleotid enthaltend, das für die Cargomolekül-Transduktionsdomäne nach Anspruch 1 oder 2 kodiert.

10. Expressionsvektor zur Expression eines rekombinanten Cargomolekülproteins mit verbesserter Zellmembranpermeabilität, wobei der Expressionsvektor das genetische Konstrukt nach Anspruch 9 umfasst.

11. Expressionsvektor nach Anspruch 10, ferner ein Gen umfassend, das für ein Cargomolekülprotein kodiert, sodass ein rekombinantes Cargomolekülprotein, in dem eine Cargomolekül-Transduktionsdomäne und das Cargomolekülprotein fusioniert sind, exprimiert werden kann.

12. Rekombinantes Cargomolekül, bei dem die Cargomolekül-Transduktionsdomäne nach Anspruch 1 oder 2 mit einem oder mehreren von einem N-Terminus und einem C-Terminus des Cargomoleküls fusioniert ist, zur Verwendung in der Therapie.

13. Rekombinantes Cargomolekül zur Verwendung nach Anspruch 12, wobei das Cargomolekül ein therapeutisches Protein ist.

14. Rekombinantes Cargomolekül zur Verwendung nach Anspruch 12, wobei das Cargomolekül ein antioxidatives Protein ist.

## Revendications

1. Domaine de transduction de molécule cargo qui se lie à des molécules cargo et transporte les molécules cargo dans des cellules ou des tissus de mammifère, le domaine de transduction de molécule cargo comprenant :
un peptide RMAD1 choisi dans le groupe constitué par SEQ ID N° : 1, SEQ ID N° : 2, SEQ ID N° : 3, SEQ ID N° : 4, SEQ ID N° : 5, SEQ ID N° : 6, SEQ ID N° : 7, SEQ ID N° : 8, SEQ ID N° : 9, SEQ ID N° : 10 et SEQ ID N° : 11 dérivées de l'ADARB2 humain.

2. Domaine de transduction de molécule cargo selon la revendication 1, dans lequel un ou plusieurs d'un peptide RMAD1 choisi dans le groupe constitué par SEQ ID N° : 1, SEQ ID N° : 2, SEQ ID N° : 3, SEQ ID N° : 4, SEQ ID N° : 5, SEQ ID N° : 6, SEQ ID N° : 7, SEQ ID N° : 8, SEQ ID N° : 9, SEQ ID N° : 10 et SEQ ID N° : 11 dérivées de l'ADARB2 humain, sont liés sous la forme d'un dimère ou d'un multimère d'ordre supérieur sans lieur ou par l'intermédiaire d'un lieur.

3. Molécule cargo recombinante présentant une perméabilité membranaire cellulaire améliorée dans laquelle une molécule cargo ; et l'un quelconque des domaines de transduction de molécule cargo choisis parmi l'une des revendications 1 ou 2 sont fusionnés à l'une ou plusieurs d'une extrémité N-terminale et d'une extrémité C-terminale de la molécule cargo.

4. Molécule cargo recombinante de la revendication 3, dans laquelle la molécule cargo est un peptide, une protéine ou un acide nucléique.

5. Molécule cargo recombinante de la revendication 3, dans laquelle la molécule cargo est une protéine thérapeutique, une protéine antigénique ou un peptide épitope.

6. Molécule cargo recombinante de la revendication 3, ladite molécule cargo étant une protéine antioxydante.

7. Médicament contenant la molécule cargo recombinante de l'une quelconque des revendications 3 à 6.

8. Produits cosmétiques contenant la molécule cargo recombinante de l'une quelconque des revendications 3 à 6.

9. Construction génique contenant un polynucléotide codant pour le domaine de transduction de molécule cargo de la revendication 1 ou 2.

10. Vecteur d'expression destiné à exprimer une protéine de molécule cargo recombinante présentant une perméabilité membranaire cellulaire améliorée, le vecteur d'expression comprenant la construction génique de la revendication 9.

11. Vecteur d'expression de la revendication 10, comprenant en outre un gène codant pour une protéine de molécule cargo de sorte qu'une protéine de molécule cargo recombinante dans laquelle un domaine de transduction de molécule cargo et la protéine de molécule cargo sont fusionnés, puisse être exprimée.

12. Molécule cargo recombinante dans laquelle le domaine de transduction de molécule cargo de l'une des revendications 1 ou 2 est fusionné à l'une ou plusieurs d'une extrémité N-terminale et d'une extrémité C-terminale de la molécule cargo, destinée à être utilisée en thérapie.

13. Molécule cargo recombinante destinée à être utilisée selon la revendication 12, ladite molécule cargo étant une protéine thérapeutique.

14. Molécule cargo recombinante destinée à être utilisée selon la revendication 12, ladite molécule cargo étant une protéine antioxydante.
